# EUROPEAN PATENT APPLICATION

(11) **EP 1 583 020 A2**
(43) Date of publication of application: **05.10.2005**
(21) Application number: 05251161.5
(22) Date of filing: 28.02.2005
(51) Int. Cl.: G06F 19/00

(54) **Program, method and device for analysis of the time-series data obtained by DNA array method**

(30) Priority: 27.02.2004 JP 2004053743
(71) Applicant: National University Corporation Nagoya University, Nagoya-city, Aichi Pref. (JP)
(72) Inventor: Ishiura, Masahiro, Nagoya City Aichi Pref. (JP); Okamoto, Kazuhisa, Nagoya City Aichi Pref. (JP)
(74) Representative: Potter, Julian Mark

(57) **Abstract**

According to this invention, a program for analysis of the time-series data obtained by DNA array method is provided, the program instructing a computer to execute a function to arrange numerical data representing the expression of genes obtained by DNA array method to a database in the order of the time-series, and a function to visualize the numerical data in the order of the time-series. Moreover, this invention enables to arrange the numerical data on gene expression in the order of the time-series and to execute statistical processing on the data, to calculate rhythm parameters by mathematical analysis, to visualize the rhythm data based on the result obtained by the analysis, and to classify the rhythm data according to plural criteria.

## Description

### BACKGROUND OF THE INVENTION

### 1. Field of the Invention

The present invention relates to a program for analysis of the time-series data obtained by DNA array method, a method for analysis of the time-series data obtained by DNA array method, and a device for analysis of the time-series data obtained by DNA array method.

### 2. Description of the Related Art

The DNA array method is an innovative technique that enables exhaustive measurement on the expression of the genes contained in the genome of an organism. However, the current status for analysis of huge volume of the data obtained by DNA array method in order of time-series is not sufficient yet.

The programs currently available for analysis of data obtained by DNA micro-array include GenePix Pro (Axon Instruments), GeneSpring (Silicon Genenetics), and etc. Use of GenePix Pro 4.1 enables one to compare two sets of DNA array data, but it is useless to process plural sets of DNA array data, such as data arranged in order of time-series.

Use of GeneSpring allows one to visualize plural sets of data from DNA arrays in order of time-series. However, operation of this program including data reading operation is very complicated and its functions, such as statistic processing and analysis of a data, clustering of the data, and output of the analyzed data, are also insufficient. Furthermore, it is quite impossible to analyze the rhythmicity of a data. Using GeneSpring, one cannot determine whether or not a given data includes some periodic fluctuation, one can not calculate rhythm parameters in the presence of certain periodic fluctuation, and one can never achieve clustering of genes according to the parameters from the analysis.

As disclosed in the Japanese Patent Application No. 2003-061203, the present inventors have developed a means for measurement and analysis of luminescence of biological samples, which enables real-time display of the measured data. According to the invention disclosed in the Japanese Patent Application No. 2003-061203, it becomes possible to assay the luminescence of biological samples arranged in a 96-well plate in real-time manner, and to print out the analyzed results. However, no attempt has been made to apply this system to processing of the data obtained from a DNA array.

### SUMMARY OF THE INVENTION

The object of the present invention is to provide, based on the newly developed means for analysis of data which is disclosed in the Japanese Patent Application No. 2003-061203, a means for alleviating the technical problem of how to put huge volume of the data obtained by DNA array method into a database in order of time-series, and to make detailed analysis of the data, such as calculation of rhythm parameters, in a rapid and simplified manner.

Therefore, an embodiment of the present invention provides a program for analysis of the time-series data obtained by DNA array method, which comprising a function to arrange numerical data representing the expression of genes obtained by DNA array method to a database in order of time-series, and a function to visualize the numerical data in order of time-series.

In another aspect, an embodiment of the present invention provides a method for analyzing time-series data obtained by DNA array method, which comprising the steps of: recording data obtained from a DNA array in a recording medium on a computer, arranging the data in a database in order of time-series, and visualizing the numerical data in order of time-series.

In yet another aspect, an embodiment of the present invention provides a device for analysis of the time-series data obtained by DNA array method, which comprising at least a recording medium for recording the data obtained from DNA array, a means for arranging numerical data representing the expression of genes obtained by DNA array method in order of time-series, and a means for visualizing the numerical data in order of time-series.

In a preferred embodiment, it is also possible to execute a function to perform mathematical analysis for statistical analysis of the numerical data arranged in a database. Furthermore, according to another embodiment, it is also possible to calculate rhythm parameters of the numerical data arranged in a database in order of time-series by mathematical analysis of the data, and to classify the rhythm data.
The means used to classify the rhythm data may include creation of a histogram, two-dimensional principal component analysis, hierarchical clustering, and etc. According to another embodiment, it is further possible to record the obtained results as a text file or a HTML format file in a recording medium, and to output the data to a printer.

Under the favor of teachings of the present invention, it becomes possible to arrange numerical data representing the expression of genes obtained by DNA array method in order of time-series, to apply statistical analysis to the arranged data, and to calculate rhythm parameters of the data by mathematical analysis of the data. Using the teachings of the present invention, it is further possible to visualize the result obtained from the above analysis, and to show relationship among the genes based on several criteria, for example, generation of a histogram, two-dimensional principal component analysis, hierarchical clustering, and etc. Using the teachings of the invention it is still further possible to record the results in a recording medium and to output a series of data to a printer. Use of the invented program allows one to execute the above-mentioned processing easily and rapidly. These features have not been attained by any conventional programs available so far and the invented program firstly achieved such effects. Then the present invention will markedly enhance the utility of the DNA array method.

### BRIEF DESCRIPTION OF THE DRAWINGS

Fig. 1 is a schematic diagram showing the outline of DNA array method.
Fig. 2 is a figure showing a main window.
Fig. 3 is a figure showing a window displaying a histogram.
Fig. 4 is a figure showing a window displaying the result of two-dimensional principal component analysis.
Fig. 5 is a figure showing a window displaying the result of hierarchical clustering.
Fig. 6 is a figure showing a window displaying the expression profile of a gene.

### DETAILED DESCRIPTION OF THE INVENTION

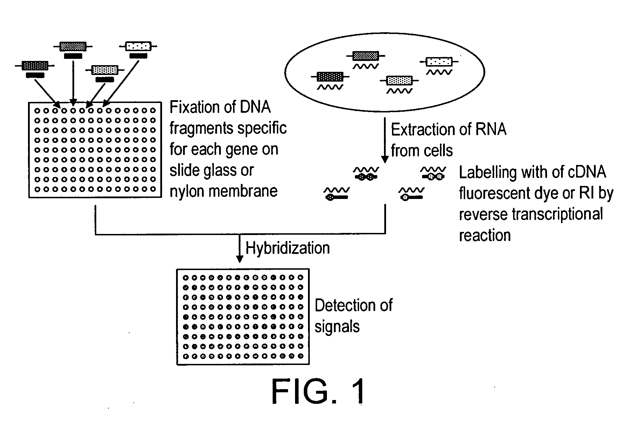

Fig. 1 is a schematic diagram showing the outline of DNA array method. DNA arrays, that consist of thousands of individual gene sequences fixed on a solid support such as a glass slide or a nylon membrane in a high-density array, provide a practical and economical tool for studying gene expression in a very large scale.
The DNA fragment fixed on the solid supports includes DNA fragments of a genome, cDNA, artificially synthesized oligonucleotides, and etc. The DNA array experiment includes following step: extraction of RNA from an organism of interest, synthesis of cDNA from the RNA using reverse transcriptase, labeling of the cDNA with a fluorescent dye or radioisotope (RI), hybridization of the labeled cDNA with DNA fragments fixed on a DNA array, and determination of the transcriptional level of genes as the intensity of fluorescence or radioactivity.

Since DNA array allows fixation of several ten thousands of different DNA on a single slide glass, and thus it is possible to investigate the expression of a huge number of genes by a single experiment. Labeled cDNA is hybridized to DNA fragments immobilized on a DNA array. Then, signals emitted from the labeled-cDNA are detected by a dedicated scanner, and the signals are converted into corresponding numerical data. The numerical data are then arranged in a database and analyzed by an analytical program, thereby the experimenter can obtain desired results.

The program to arrange the numerical data obtained by DNA array method in a database in order of time-series, and to perform detailed analysis of the data in a rapid and simplified manner. Particularly, if the time-series data contains periodic fluctuations, one can efficiently perform data analysis based on the program disclosed in the Japanese Patent Application No. 2003-061203.

Embodiments of the present invention are performed based on the Japanese Patent Application No. 2003-061203, and will provide one or more of the following six functions in addition to those disclosed in the Japanese Patent Application No. 2003-061203. (1) A function to arrange a huge amount of the numerical data obtained by DNA array method in a database in order of time-series. (2) A function to visualize a huge amount of the numerical data that represent the expression of genes in order of time-series. (3) A function to apply statistical analysis on the numerical data in order to judge whether the data significantly satisfies the criteria set by the experimenter. (4) A function to calculate rhythm parameters by mathematically analysis from the data arranged in order of time-series, (5) A function to classify the rhythm data (generation of a histogram, two-dimensional principal component analysis, and hierarchical clustering). (6) A function to record all the results in a recording medium as a text file or HTML format file and to output the data to a printer.

The program disclosed herein can also be applied for the analysis of data of bioluminescence measured as described in the Japanese Patent Application No. 2003-061203. Thus, using the present invention, it is possible to compare the data obtained by the bioluminescence measurement with those obtained by DNA array method. The characteristic features of the presently preferred embodiments are detailed below.

### (1) A function to arrange data in a database

The program is launched, and a numerical data file from DNA array experiment, which is recorded in advance in a recording medium on a computer, is designated. Then, necessary conditions are entered such as the number of the sets of numerical data files, time interval between time point numerical data, number of the DNA arrays used at each time point, number of the genes spotted on each DNA array, and the set number of the spotted genes contained in the array, and then input of the numerical data file is executed.
The program calculates the average and standard deviation of the data according to the above-mentioned conditions specified by the experimenter, arranges the results of the individual genes in arrange the results to a database in order of time-series, and records the results.

### (2) A function to calculate rhythm parameters

Data arranged in a database in order of time-series are mathematically analyzed by the method described in the Japanese Patent Application No. 2003-061203. If the method is applied for the analysis of the time-series data obtained by DNA array method, it will be possible to calculate a periodic fluctuation contained in the time-series data. Despite that many biological phenomena are periodically regulated, analysis on the periodicity can not be performed by conventional programs. By utilizing the method described in the Japanese Patent Application No. 2003-061203, it becomes possible to analyze the periodic fluctuation in data obtained by DNA array method, which has never been attained by any conventional program.

### (3) A function to determine by statistical analysis whether or not the data fulfills certain criteria

One can determine whether given temporal data set shows significant rhythmicity by mathematical analysis. Expression data corresponding to the peak and trough time points of the cycle are obtained based on the period length of the rhythm calculated by mathematical analysis, and these data are subjected to t-test to calculate the p-value (indicator for significance of difference).
The program determines that the data contains a significant rhythmic fluctuation, if the period length calculated by mathematical analysis, the residue between the original numeral data and the results calculated by mathematical analysis, and the p-value of the t-test meets the criteria set by the user. The user can set the criteria optionally: for example, when the user judges whether the data has a circadian rhythm, he may set the criteria as follows: the calculated period length is between 18.0 and 26.8 hour, the residue is 0.75 or lower, and the p-value of the t-test is 0.05 or lower.

### (4) A function to visualize numerical data

The invented program can visualize the temporal change in average expression levels of individual genes, accompanied by standard deviations. The program also allows the user to confirm the results of the mathematical analysis. Furthermore, the user can print all the analyzed results, and output them into a text file.

### (5) A function to classify rhythm data

The rhythm parameters determined by this program include period length, phase, amplitude, and significance of rhythmicity. Using the histogram function of the program, the relationship between any one parameter of rhythm and the number of genes can be plotted as a histogram. Thus, relationship between the rhythm parameters and the number of genes can be easily found. The histogram may consist of, for example, a circle whose circumference represents the hour of a day from 0 to 24th hour upon which the number of genes exhibiting peak expression for each hour is plotted. The display can also represent the name of the genes as well. By referring to the histogram, the user can easily comprehend at which time of the day which genes are most abundantly expressed.

The present program includes two-dimensional principal component analysis function. Using this function, any two rhythm parameters of the expression rhythm of each gene obtained from the analysis can be displayed as a two-dimensional scatter plot. Therefore, the user can easily determine whether there is any relationship between the rhythm parameters.

The present program also includes hierarchical clustering function. Using this function, the program can classify genes according to multiple rhythm parameters. Thus, the user can easily predict on (1) whether or not the genes classified into a group have functional relationship with each other, (2) the function of a gene whose function remains unknown, and (3) the mechanism of regulation of gene expression on a gene whose mechanism working for it remains unknown. In contrast, since conventional programs can not calculate periodicity contained in DNA array data, it is not possible to classify genes on the basis of rhythm parameters. The most characteristic feature of the invented program is that it enables such function that has never been attained by any conventional programs.

Embodiments of the present invention are further detailed below with reference to Examples, but those examples do not limit in any way the scope of the present invention.

### EXAMPLES

The expression of 3070 genes obtained by DNA array method from bacterial species *Synechocystis* was analyzed using the invented program. Wild-type cells of *Synechocystis* sp. strain PCC 6803, were inoculated on BG-11 liquid medium, and they were grown with aeration at 30°C under 90 µmol.m⁻².sec⁻¹ white light illumination (constant light condition). When the OD₇₃₀ of the culture medium reached to 0.35 (3.5 × 10⁸ cells/ml), BG-11 liquid culture medium was added to maintain the density of cells at OD₇₃₀ value of 0.35 (3.5 × 10⁸ cells/ml), using a continuous cultivation apparatus. To reset the biological clock, the cells were kept in darkness for 12 hours. After the 12-hour dark period, the cells were returned to the constant light condition.
The time of completion of the 12-hour dark period was defined as 0 hour, and aliquots were collected from the culture at every 4-hour during the period between 0 hour and 48 hour. The aliquot culture medium was rapidly frozen with liquid nitrogen, and stored at -85°C until extraction of RNA. The extraction of RNA was conducted by phenol/SDS method.

DNA array of *Synechocystis* sp. strain PCC 6803, CynanoCHIP ver 1.6 (Takara, Japan) was used. On this DNA array, two sets of 3070 DNA fragments which essentially cover all of the genes contained in the *Synechocystis* were fixed. At each time point after transfer to constant light, aliquots were taken from the culture of *Synechocystis* and 5 µg of total RNA was prepared. Then cDNA labeled with a fluorescent dye was synthesized using a fluorescent label core kit (M-MLV ver 2.0, Takara, Japan). Cy3-dUTP or Cy5-dUTP (Amersham, UK) was incorporated during synthesis of the first cDNA strand with random primer. Cy3-labeled test cDNA was synthesized with total RNA from cells harvested at each time point. Cy5-labeled reference cDNA was synthesized with a mixture of total RNA sample from all time points.

These cDNAs were competitively hybridized on a microarray. Hybridization was carried out for 16 h at 65°C in 20 µL 6 X SSC (1 X SSC is 0.15 M NaCl, 0.015 M sodium citrate), 0.2% SDS, 5 X Denhardt's solution, and 100 ng/µL denatured salmon sperm DNA. After hybridization, the microarrays were washed with 2 X SSC, 0.2% SDS once at 55°C for 5 min and twice at 65°C for 5 min, and then rinsed with 0.05 X SSC at room temperature. The washed microarrays were dried by centrifugation. Fluorescence images of Cy3 and Cy5 were obtained with a GenePix 4000B scanner (Axon Instruments, CA). Each microarray was scanned twice. The second scan was performed with lower photo multiplier tube gain to avoid signal saturation.

The signal intensity of each spot and its local background were determined with GenePix Pro software (Axon Instruments, CA). Net signal intensity was calculated by subtraction of the median signal intensity of all pixels within the local background area from the median signal intensity of all pixels within the spot area. Correct recognition of all spot areas by the automatic alignment function of the GenePix Pro was confirmed visually. Spots meeting any of following criteria were flagged and not used for subsequent data analysis: (i) the GenePix Pro did not find the spot area automatically, (ii) the net signal intensity was ≤ 0, (iii) the percentage of saturated pixels in the spot area was ≥ 25, and (iv) severe noise was present. Biases in signal intensity between the two fluorescent dye channels in a microarray were normalized by locally weighted linear regression analysis (lowess normalization) using MIDAS (freely available from http://www.tigr.org/software/tm4/midas.html). For all normalization, the smoothing parameter was set to 0.33. The normalized data will be available at http://www.genome.ad.jp/kegg/expression/. The relative expression level of a gene at a time point was calculated as log2 (Cy3/Cy5), where Cy3 and Cy5 were normalized signal intensities from test and reference cDNA. The mean of the relative expression levels from the three technical replicates was calculated for each biological replicate and used in the subsequent analysis. Genes carrying fewer than two unflagged data at any time point were removed from the analysis. We calculated ratio of net signal intensity to background standard deviation of all spots for each gene, and genes with average ratio ≥ 2.5 were considered as detectably expressed (2,648 genes). Genes that did not satisfy this criterion were not analyzed further. The relative expressions of a gene at each time point were calculated by the formula of log₂(Cy3/Cy5). The relative expression values was recorded on a computer for each DNA array as a text file. Then the average and standard deviation of relative expression of each genes at each time points were calculated using the invented program from the data, and the results were put to a database in order of time-series.

Using the method described in the Japanese Patent Application No. 2003-061203, the data was arranged in a database in order of time-series and applied to mathematical analysis for statistical study, and the p-value was calculated using t-test. In this case, it was concluded that the data shows significant circadian rhythm, if the period length obtained by mathematical analysis is between 18.0 and 26.8 hour, the residue between the original numeral data and the simulated values calculated from mathematical analysis is 0.75 or lower, and the p-value for the t-test is 0.05 or lower. Then, genes that satisfy the above-mentioned criteria were searched using the search function described in the Japanese Patent Application 2003-061203. Then, it was found that 644 of the 3,070 genes searched exhibit significant circadian rhythm in their gene expression.

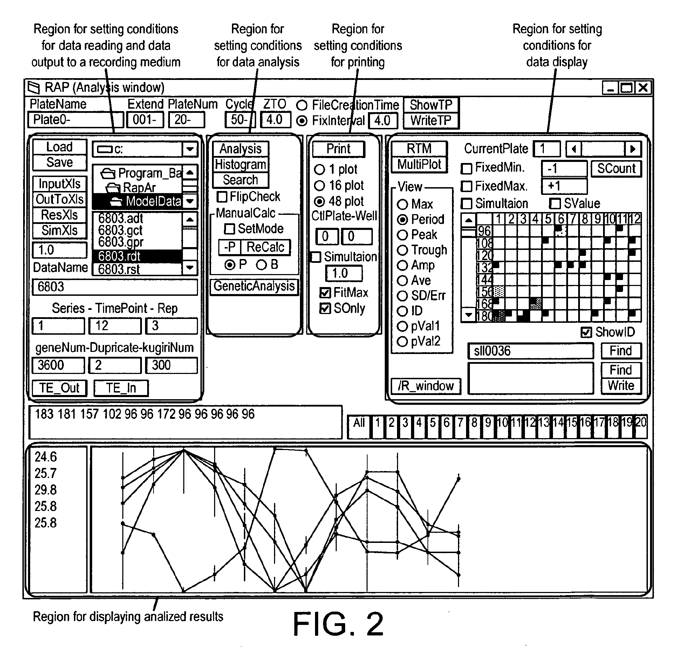

Fig. 2 shows a main window, which is used for numerical data reading and analysis of these data. From this window, subwindow for executing each function was started up. The upper panel of the window shown in Fig. 2 represents, from left to right, the region for setting conditions for the reading of data and conditions for output of the data to a recording medium, the region for setting conditions for the analysis of data, the region for setting conditions of printing, and the region for setting conditions for the displaying the data, respectively. The lower panel of the figure represents the region for displaying analyzed results. In Fig. 2, the expression level of each gene was visualized on the program in order of time-series, as the average and standard deviation at each point. Moreover, the result of mathematical analysis could be confirmed visually on the program as well. Furthermore, output of all of the analyzed results was achieved as a text file or a printing.

Moreover, by the histogram function of the inventive program, it is possible to illustrate the rhythm parameters of gene expression rhythm of each gene derived from the analyzed result.

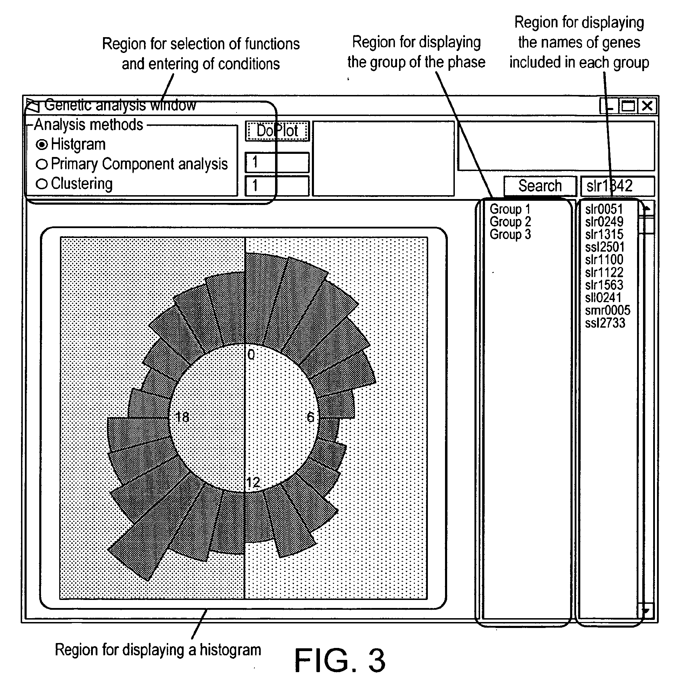

Fig. 3 shows a window that displays a histogram representing the peak hour of the rhythm of gene expression and the number of genes in the 644 genes (which were found to exhibit circadian rhythm at a significant level) derived from *Synechocystis* sp. strain PCC 6803 as a histogram. Namely, Fig. 3 illustrates the peak hour (phase) in the rhythm of gene expression and the number of genes, as a histogram. The upper panel of Fig. 3 represents the region for selection of functions and the region for entering conditions. The lower panel of Fig. 3 represents, from left to right, the region for displaying a histogram, the region for displaying the group of the phase, and the region for displaying the name of genes included in each group, respectively.

In the histogram in Fig. 3, the hour in the range of 0 hour to 24 hour along the circumference of a circle and the number of genes that exhibit the peak expression at each hour are represented as a histogram, and the names of the genes corresponding to each group are represented in the right-hand box. Hereby the user can easily comprehend which gene is expressed at which hour of a day. With regard to *Synechocystis* sp. strain PCC 6803, the majority of its genes exhibit maximum expression at the early morning and at the early evening. It is also possible to identify which gene exhibits its peak expression for each hour of a day.

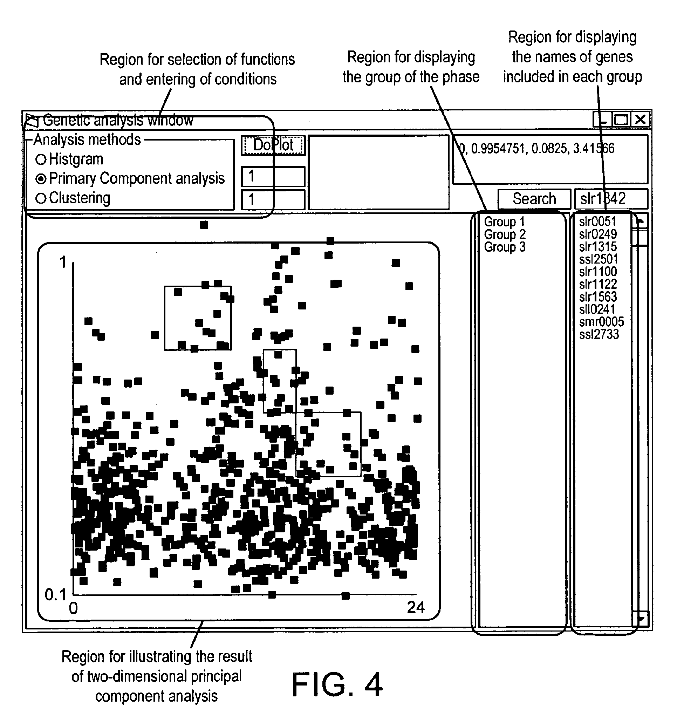

As described above, according to the two-dimensional principal component analysis function provided to the inventive program, it is possible to illustrate any two kinds of rhythm parameters of the expression rhythm of each gene obtained from analyzed result, as a two-dimensional scatter-plot. For the 644 genes of *Synechocystis* sp. strain PCC 6803 (which were found to exhibit circadian rhythm at a significant level), the hour (phase) at which the genes exhibit the peak expression in the expression rhythm and the amplitude of the rhythm of gene expression were represented as a two-dimensional scatter-plot. The result is shown in Fig. 4. In Fig. 4, the left upper portion represents the region for the selection of desired function and entering of conditions, the left lower portion represents the region for illustrating the result of a two-dimensional principal component analysis, the central lower portion represents the region for displaying the group of phase, and the right lower portion represents the region for displaying the name of genes included in each group, respectively. From the result in Fig. 4, it was revealed that, there is no relationship between the phase of the gene expression rhythm and the amplitude of the rhythm in genes of *Synechocystis* sp. strain PCC 6803.

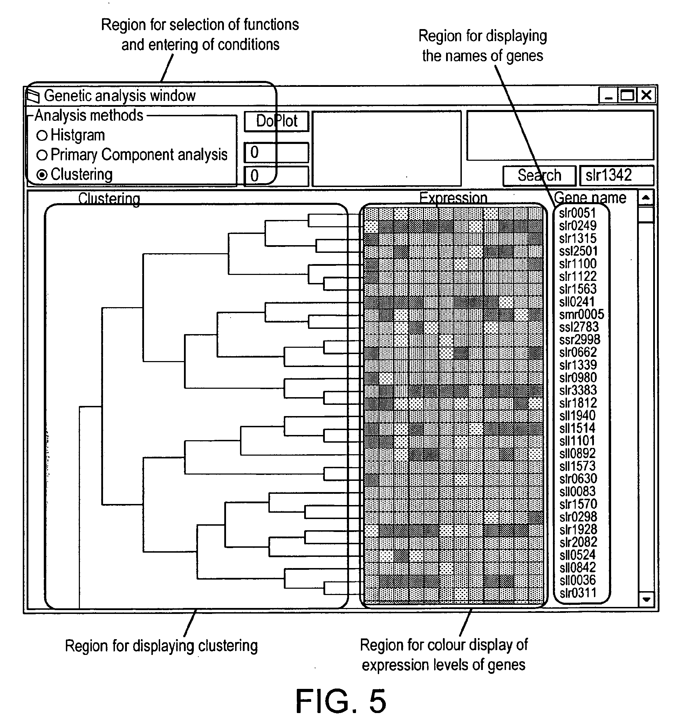

Furthermore, utilizing the hierarchical clustering function provided to the inventive program, each gene was classified into groups using rhythm parameters obtained from the analyzed result as criteria. As mentioned above, by performing the hierarchical clustering, one can easily determine whether or not there is any functional relationship among genes classified into each group.
The hierarchical clustering was performed using the peak hour (phase) of the expression rhythm of 644 genes of *Synechocystis* sp. strain PCC 6803 (which was found to exhibit a circadian rhythm at a significant level) as criteria, and the result is shown in Fig. 5. In Fig. 5, the phenotypes of genes were classified on the basis of the relationship of rhythm parameters obtained from the analysis, and the expression pattern is represented by pseudo-color with the figure of clustering. From Fig. 5, it was revealed that genes with close functional relationship tend to express in the groups of closely related phase in regard to genes of *Synechocystis* sp. strain PCC 6803.

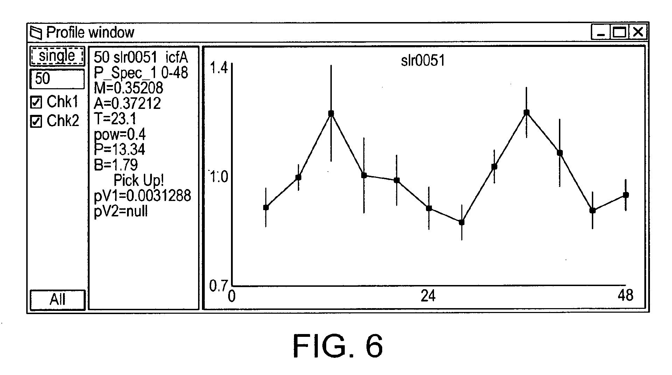

Fig. 6 represents a window showing the expression profile of a gene. The user can display the window of expression profile of a gene by selecting the name of the gene displayed in the window for executing each function. The window also lists the information on a gene (name of the gene, graph of its expression pattern in order of time-series, average and standard deviation of the relative values of gene expression at each hour, period, phase, amplitude, and etc.).

In this Example, for each of 12 time points, three DNA arrays were treated (36 arrays in total), and numerical data obtained from the DNA array were processed using the inventive program.
Since each DNA array contains two spots for each gene, it is estimated that 221,040 numerical data were processed in total. The inventive program enabled to arrange immense number of numerical data obtained in the Example to a database in order of time-series, and to visualize the data in order of time-series. The inventive program further enabled mathematical analysis of numerical data arranged to a database in order of time-series for statistical processing, and determination whether or not the result meets the criteria set by the experimenter with significance.

As discussed in connection with the above Examples, according to the inventive program, it was possible to arrange numerical data obtained by DNA array method in order of time-series, and to calculate the rhythm parameters of the data by mathematical analysis. It was also possible to visualize the rhythm data on the basis of analyzed results, to classify the rhythm data obtained on the basis of plural criteria, and to perform analysis such as creation of histogram, two-dimensional principal analysis and hierarchical clustering. It was still further possible to record a series of results thus obtained on a recording medium, and to output them to a printer.
Use of the inventive program alone allowed the user to achieve the series of processing as described above in a rapid and simplified manner. This feature has never been achieved by any conventional programs so far and is characteristic for the program described herein, and this program will enhance the utility of the DNA array method markedly.

The program described herein allows systematic analysis of a huge amount of DNA array data in order of time-series.
Thus, it is likely that the inventive program will markedly enhance the utility of DNA array method. The programs for data analysis that promotes usefulness of DNA array method have been enthusiastically demanded in the market of biological field. Thus, it is assumed that the program described herein may be attached to a commercially available device for DNA array when the device is sold or it may be sold by itself separately.

Whilst certain preferred embodiments of the invention have been described herein in detail, it should be noted that modifications may be made to those embodiments without departing from the scope of the present invention.

Viewed differently, in general terms, an embodiment of the present invention provides a computer program comprising computer program elements for implementing at least part of the method claimed in claims 10 to 18 hereafter. Another embodiment of the present invention, again viewed differently in general terms, provides a device for analysis of the time-series data obtained by DNA array method, the device being configured to implement the method of claims 10 to 18 hereafter, and comprising: a recording medium for recording the data obtained from DNA array, a means for arranging numerical data representing the expression of genes obtained by DNA array method in order of time-series, and a means for visualizing the numerical data in order of time-series.

It will additionally be appreciated that the computer program herein described may be embodied as source code and undergo compilation for implementation of a computer, or may be embodied as object code, for example. The computer program, or aspects of the program, may even be embodied as hardware, for example one or more application specific integrated circuits.

Furthermore, whilst the accompanying claims define certain combinations of features, it should be noted that the scope of the present invention is not limited to the particular combination of features set out in the accompanying claims but instead extends to any combination of features described herein.

## Claims

1. A program for analysis of time-series data obtained by DNA array method, which comprising a function to arrange numerical data representing the expression of genes obtained by DNA array method to a database in order of time-series, and a function to visualize the numerical data in order of time-series.

2. The program as described in Claim 1 further instructing a computer to execute a function of mathematical analysis and statistical processing on the numerical data arranged to a database by executing the function to arrange the numerical data to a database in order of time-series.

3. The program as described in Claim 1 or 2 further instructing a computer to execute a function to calculate a rhythm parameters by mathematical analysis on the numerical data arranged to a database by executing the function to arrange the numerical data to a database in order of time-series.

4. The program as described in any one of Claims 1 to 3 further instructing the computer to execute a function to classify rhythm data obtained by mathematical analysis on the numerical data arranged to a database by executing the function to arrange the numerical data to a database in order of time-series.

5. The program as described in Claim 4 wherein the means for classifying rhythm data is creation of a histogram, two-dimensional principal component analysis, or hierarchical clustering.

6. The program as described in Claim 5 wherein the means for classifying rhythm data is creation of a histogram, thereby enables analysis of the expression pattern of a gene in order of time-series.

7. The program as described in Claim 5 wherein the means for classifying rhythm data is two-dimensional principal component analysis, thereby enables illustration of the relationship between any two kinds of rhythm parameters in a two-dimensional scatter-plot.

8. The program as described in Claim 5 wherein the means for classifying rhythm data is hierarchical clustering, thereby enables classification of genes into groups using any plural kinds of rhythm parameters as criteria.

9. The program as described in any one of Claims 1 to 8 further instructing the computer to execute a function to record the obtained results as a text file or a HTML format file in a recording medium, and to output the file or the data to a printer.

10. A method for analyzing time-series data obtained by DNA array method, which comprising the steps of: recording numerical data representing the expression of genes obtained from a DNA array in a recording medium on a computer, arranging the data to a database in order of time-series, and visualizing the numerical data in order of time-series.

11. The method as described in Claim 10 further comprising the step of directing a computer to execute a function of mathematical analysis and statistical processing on the numerical data arranged to a database by executing the function to arrange the numerical data to a database in order of time-series.

12. The method as described in Claim 10 or 11 further comprising the step of directing a computer to execute a function to calculate a rhythm parameters by mathematical analysis on the numerical data arranged to a database by executing the function to arrange the numerical data to a database in order of time-series.

13. The method as described in any one of Claims 10 to 12 further comprising the step of directing a computer to execute a function to classify rhythm data obtained by mathematical analysis of the numerical data arranged to a database as a result of the execution of the function to arrange the numerical data to a database in order of time-series.

14. The method as described in Claim 13 wherein the means for classifying rhythm data is creation of a histogram, two-dimensional principal component analysis, or hierarchical clustering.

15. The method as described in Claim 14 wherein the means for classifying rhythm data is creation of a histogram, thereby enables analysis of the expression pattern of a gene in order of time-series.

16. The method as described in Claim 14 wherein the means for classifying rhythm data is two-dimensional principal component analysis, thereby enables illustration of the relationship between any two kinds of rhythm parameters to a two-dimensional scatter plot.

17. The method as described in Claim 14 wherein the means for classifying rhythm data is hierarchical clustering, thereby enables classification of genes into groups using any plural kinds of rhythm parameters as criteria.

18. The method as described in any one of Claims 10 to 17 further comprising the step of directing the computer to execute a function to record the obtained results as a text file or a HTML format file in a recording medium, and to output the file or the data to a printer.

19. A device for analysis of the time-series data obtained by DNA array method, which comprising at least a recording medium for recording the data obtained from DNA array, a means for arranging numerical data representing the expression of genes obtained by DNA array method in order of time-series, and a means for visualizing the numerical data in order of time-series.

20. The device as described in Claim 19 further comprising a means for directing a computer to execute a function of mathematical analysis and statistical processing on the numerical data arranged to a database by executing the function to arrange the numerical data to a database in order of time-series.

21. The device as described in Claim 19 or 20 further comprising a means for directing a computer to execute a function to calculate rhythm parameters by mathematical analysis on the numerical data arranged to a database by executing the function to arrange the numerical data to a database in order of time-series.

22. The device as described in any one of Claims 19 to 21 further comprising a means for directing a computer to execute a function to classify the rhythm data obtained by mathematical analysis of the numerical data arranged to a database, as a result of the execution of the function to arrange the numerical data in order of time-series.

23. The device as described in Claim 22 wherein the means for classifying rhythm data is creation of a histogram, two-dimensional principal component analysis, or hierarchical clustering.

24. The device as described in Claim 23 wherein the means for classifying rhythm data is creation of a histogram, thereby enables analysis of the expression pattern of a gene in order of time-series.

25. The device as described in Claim 23 wherein the means for classifying rhythm data is two-dimensional principal component analysis, thereby enables illustration of the relationship between any two kinds of rhythm parameters to a two-dimensional scatter plot.

26. The device as described in Claim 23 wherein the means for classifying rhythm data is hierarchical clustering, thereby enables classification of genes into groups using any plural kinds of rhythm parameters as criteria.

27. The device as described in any one of Claims 19 to 26 further comprising the means for instructing the computer to execute a function to record the obtained results as a text file or a HTML format file in a recording medium, and to output the file or the data to a printer.
